(19) Europäisches Patentamt
European Patent Office
Office européen des brevets

(11) Publication number: 0 246 071 A2

# (12) EUROPEAN PATENT APPLICATION

(21) Application number: **87304218.8**

(22) Date of filing: **12.05.87**

(51) Int. Cl.[4]: **C 07 C 131/00**
A 01 N 37/50

(30) Priority: **16.05.86 US 864046**

(43) Date of publication of application:
**19.11.87 Bulletin 87/47**

(84) Designated Contracting States:
**AT BE CH DE ES FR GB GR IT LI LU NL SE**

(71) Applicant: **Rohm and Haas Company**
**Independence Mall West**
**Philadelphia, Pennsylvania 19105 (US)**

(72) Inventor: **Chi-Tung-Hsu, Adam**
**1686 Heebner Way**
**Lansdale Pennsylvania 19446 (US)**

(74) Representative: **Angell, David Whilton et al**
**ROHM AND HAAS (UK) LTD. European Operations Patent Department Lennig House 2 Mason's Avenue**
**Croydon CR9 3NB (GB)**

## (54) Alpha-halopyruvate oxime.

(57) Alpha-halopyruvate oximes of the formula:

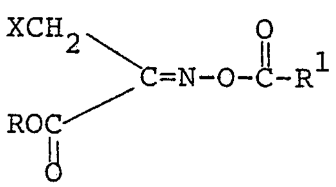

wherein R, $R^1$, and X are as defined herein have bactericidal and/or fungicidal activity. Certain of these oximes are novel compounds.

EP 0 246 071 A2

**Description**

## ALPHA-HALOPYRUVATE OXIME

This invention is concerned with the use of certain alpha-halopyruvate oximes as bactericides and fungicides, and with some of these oximes which are novel compounds and compositions containing these novel compounds.

The alpha-halopyruvate oximes which are useful bactericides and/or fungicides (I, infra) are represented by the formula:

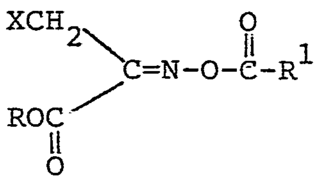

I

wherein X is halo: R is alkyl, for example, $(C_1-C_6)$alkyl (methyl, ethyl, propyl, butyl, pentyl, hexyl: $R^1$ is alkyl, for example, straight or branched chain $(C_1-C_{15})$alkyl; haloalkyl, for example, mono-, di- or trihalo $(C_1-C_6)$alkyl; alkenyl, for example, $(C_2-C_6)$alkenyl; haloalkenyl, for example, halo-$(C_2-C_6)$alkenyl; alkoxy for example, $(C_1-C_6)$alkoxy; haloalkoxy; alkoxy carbonyl; alkoxy carbonylalkyl; aryl, aryloxy or aryloxyalkyl, for example, mononuclear and dinuclear aryl or aryloxy; substituted aryl, aryloxy or aryloxyalkyl wherein the substituents are selected from one or more halo, $(C_1-C_6)$alkyl, $(C_1-C_6)$alkoxy, halo$(C_1-C_6)$alkyl or nitro substituents, aralkyl, for example, aryl-$(C_1-C_6)$alkyl and the like, arylalkenyl, for example, aryl-$(C_2-C_6)$ alkenyl and the like, cycloalkyl, for example, cyclo$(C_3-C_7)$alkyl or $(C_1-C_6)$alkylamino.

Halo includes chloro, fluoro, bromo and iodo, aryl includes mononuclear aryl of from 6 to 10 nuclear carbon atoms such as phenyl and naphthyl, haloalkyl includes mono-, di- and trihaloalkyl groups such as chloromethyl, dichloromethyl, trifluoromethyl, dibromomethyl and 1,2-dichloroethyl; alkyl includes methyl, ethyl, propyl, butyl, hexyl, heptyl, octyl, nonyl, decyl, undecyl, dodecyl and their isomers such as tert-butyl and iso-propyl; cycloalkyl includes cyclopropyl, cyclobutyl, cyclopentyl and cyclohexyl; alkenyl includes ethenyl, propenyl, 1-butenyl, 2-butenyl, 2-methylpropenyl, 3,3-dimethyl-1-butenyl, 4-methyl-2-pentenyl, allyl, 1,3-butadiene, 1,4-pentadiene, 1,3,5-hexatriene, isopropenyl and 1-isobutenyl; haloalkenyl includes chloroallyl and the like; alkoxy includes methoxy, ethoxy, propoxy and butoxy; aryloxy includes phenoxy and naphthyloxy; substituted aryl, aryloxy or aryloxyalkyl includes substituted phenyl and phenoxy such as 2, 3 or 4-bromophenyl or phenoxy; 2-, 3- or 4-trifluoromethylphenyl or phenoxy, 2,3 or 4-methyl- or methoxyphenyl or phenoxy; 2,4-, 2,6-, 3,4- or 3,6-dichlorophenyl or phenoxy; 2,6-difluorophenyl or phenoxy; 3 or 4-chloromethylphenyl or phenoxy, 4-cyanophenyl or phenoxy; 4-nitrophenyl or phenoxy, 4-chlorophenoxymethyl, 4-tert-butylphenyl or phenoxy; 2-acetyloxyphenyl or phenoxy; aralkyl includes benzyl, phenethyl, alpha-methylbenzyl, 3-phenylpropyl and 2-phenylpropyl; aralkenyl includes styrene, cinnamyl(1-phenylpropene), 3-phenylpropene, 2-phenyl-2-butene, 4-phenyl-2-butene, 4-phenylbutene, 5-phenyl-1,3-pentadiene, 5-phenyl-1,4-pentadiene and 3-phenyl- 1,2-propadiene and $(C_1-C_6)$alkylamino includes methylamino, ethylamino and propylamino.

Primarily because of their excellent bactericidal and fungicidal activity, compounds of the following formula are preferred for use according to the invention:

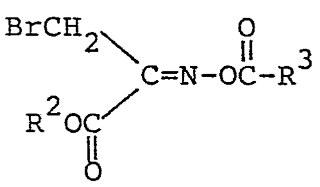

Ia

wherein $R^2$ is $(C_1-C_6)$alkyl and $R^3$ is $(C_1-C_6)$alkyl, $(C_2-C_6)$alkenyl, phenyl-$(C_1-C_6)$alkyl, halophenyl, trifluoromethylphenyl, nitrophenyl or methylamino.

All of the compounds of Formula I (supra) (except when $R^1$ is alkylamino) may be prepared by the following process:

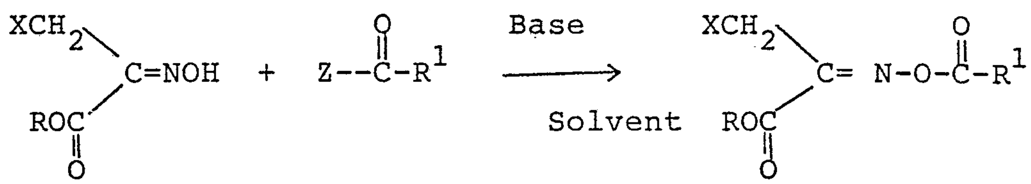


II III Ib

where R, $R^1$, X are as defined above and Z is halo, preferably chloro.

This reaction may be carried out at a temperature in the range of from about 0° C to about 80° C. Generally, the reaction is exothermic so in most cases no heating is required. The products of Formula Ib can be isolated by known methods. Suitable solvents for conducting the reaction include inert organic solvents such as toluene, chloroform, methylene chloride and carbon tetrachloride. Preferred bases for the reaction are organic tertiary bases such as triethylamine and pyridine, or inorganic bases such as sodium bicarbonate and potassium hydrocarbonate.

Those compounds wherein $R^1$ is alkylamino can be prepared by treating the compound of Formula II (supra) with an isocyanate (O=C=N-alkyl).

The compounds of Formula II (supra) can be prepared by the following procedure:

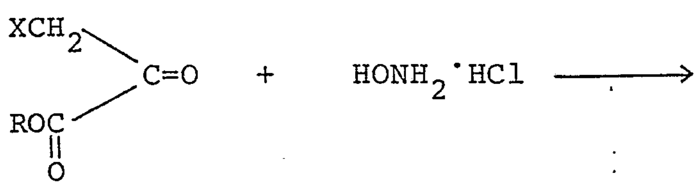

This reaction may be conducted in an alkaline solution with or without mild heating.

The above compounds of the invention can readily be utilized as bactericides and fungicides or combinations thereof in any locus, such as, for example, paper pulp processes, aqueous polymer dispersions, water-based paints, seed treatment applications and the like. In addition, these compounds and compositions containing them can function as fabric or leather preservatives, wood preservatives, cosmetic preservatives, soap additives, sanitizing agents, such as in laundry soaps and detergents, and preservatives for metal working compounds, such as emulsifiable cutting oils, preservatives for fuels or fiber spin finish biocides.

The invention also extends to those of the compounds of formula I which are novel compounds. These are those in which

X is halo;

R is alkyl;

$R^1$ is haloalkyl, haloalkenyl, haloalkoxy, ($C_1$-$C_6$)alkylamino or aryl substituted with one or more halo, haloalkyl or nitro.

Preferred novel compounds are those in which one or more of the substituents conforms to the following definitions:

Y is bromine; R is ($C_1$-$C_{15}$)alkyl; $R^1$ is halo ($C_1$-$C_6$)alkyl, halophenyl, trifluoromethylphenyl, nitrophenyl or methylamino.

Especially preferred options for R are methyl and ethyl and for $R^1$ are chloro methyl, 2-, 3- or 4-chlorophenyl, 2-, 3- or 4-methylphenyl, 2-, 3- or 4-fluorophenyl and 2,4- or 2,6-dichlorophenyl.

Most preferred are the compounds in which X is bromine, R is ethyl and $R^1$ is 4-chlorophenyl, 4-methylphenyl, 3-methylphenyl, propyl, 1-methylethenyl, 2-phenylethenyl or 4-fluorophenyl.

The invention further extends to bactericidal and/or fungicidal compositions comprising at least one of the novel compounds together with environmentally acceptable diluent or carrier.

In general, a locus subject to contamination by microorganisms can be protected in accordance with this invention by treatment with an alpha-halopyruvate oxime in an amount which is effective to control the microorganisms. The term "contamination" is meant to include any attack by microorganisms which leads to a chemical or physical breakdown or disintegration of the locus as well as the proliferation of the microorganisms within or at the locus without an accompanying deleterious effect. The exact amount of alpha-halopyruvate oxime required will, of course, vary with the medium being controlled, the particular alpha-halopyruvate oximes or compositions containing the alpha-halopyruvate oxime being employed and other factors. Typically, for treatment of a liquid medium, excellent control is obtained when the oximecarbamate is incorporated in an amount of from about 0.01 to about 10,000 parts per million (ppm) or up to 95% based on the weight of the composition. A range of from about 0.05 to about 2,500 ppm is preferred.

The term "control" as employed in the specification and claims of this application is construed to mean any adverse affect on the existence, establishment or growth of any living organism or microorganism. This effect may comprise a complete killing action, eradication, arresting in growth, inhibition, protection, reduction in number or any combination thereof.

The alpha-halopyruvate oximes of Formula I are useful as agricultural fungicides. As such, they are particularly valuable when formulated in a fungicidal composition. Such compositions normally comprise an agronomically acceptable diluent or carrier and a alpha-halopyruvate oxime or mixture of alpha-halopyruvate oximes as the active agent. Where necessary or desirable, surfactants or other additives may be incorporated to give uniformly formulated mixtures. "Agronomically acceptable diluent or carrier" includes any substance which can be utilized to dissolve, dispense or diffuse the chemical incorporated therein without impairing the effectiveness of the toxic agent and which does no permanent damage to such environment as soil, equipment and agronomic crops.

For use as agricultural fungicides, the compounds are usually taken up on an agronomically acceptable carrier or formulated so as to render them suitable for subsequent dissemination. For example, the alpha-halopyruvate oximes can be formulated as wettable powders, emulsion concentrates, dusts, granular formulations, aerosols or flowable emulsifiable concentrates. In such formulations the alpha-halopyruvate oxime is extended with a liquid or solid carrier and, when desired, suitable surfactants are likewise incorporated.

Compounds of this invention can be dissolved in a water-miscible liquid, such as ethanol, isopropanol and acetone. Such solutions are easily extended with water.

The alpha-halopyruvate oximes can be taken up on or mixed with a finely particled solid carrier as, for example, clays, inorganic silicates, carbonates, and silicas. Organic carriers can also be employed. Dust concentrates are conveniently made wherein alpha-halopyruvate oximes are present in the range of from about 20% to about 80% by weight. For ultimate applications, these concentrates are conveniently extended with additional solids from about 1% to about 20% by weight.

Wettable powder formulations can be made by incorporating the compounds in an inert, finely divided solid carrier along with a surfactant which may be one or more emulsifying, wetting, dispersing or spreading agents or blend of these. The alpha-halopyruvate oximes are usually present in the range of from about 10% to about 80% by weight and the surfactants are from about 0.5% to about 10% by weight. Commonly used emulsifying and wetting agents include polyoxyethylated derivatives of alkylphenols, fatty alcohols, fatty acids, and alkylamines, alkylarene sulfonates and dialkyl sulfosuccinates. Spreading agents inlcude such materials as glycerol mannitan laurate and a condensate of polyglycerol and oleic acid modified with phthalic anhydride. Dispersing agents include such materials as the sodium salt of the copolymer of maleic anhydride and an olefin such as diisobutylene, sodium lignin sulfonate and sodium formaldehydenaphthalene sulfonates.

One convenient method for preparing a solid formulation is to impregnate the alpha-halopyruvate oxime toxicant onto the solid carrier by means of a volatile solvent, such as acetone. In this manner adjuvants, such as activators, adhesives, plant nutrients, synergists and various surfactants, can also be incorporated.

Emulsifiable concentrate formulations can be prepared by dissolving the compounds in an agronomically acceptable organic solvent and adding a solvent-soluble emulsifying agent. Suitable solvents are usually water-immiscible and may be found in the hydrocarbon, chlorinated hydrocarbon, ketone, ester, alcohol and amide classes of organic solvents. Mixtures of solvents may be employed. The surfactants useful as emulsifying agents may constitute from about 0.5% to about 10% by weight of the emulsifiable concentrate and may be anionic, cationic or non-ionic in character. Anionic surfactants include alcohol sulfates or sulfonates, alkylarene sulfonates and sulfosuccinates. Cationic surfactant include fatty acid alkylamine salts and fatty acid alkyl quaternaries. Nonionic emulsifying agents include alkylene oxide adducts of alkylphenols, fatty alcohols, mercaptans and fatty acids. The concentration of the active ingredients may vary from about 10% to about 80%, preferably in the range of from about 25 to about 50%.

For use as a fungicidal agent, active compounds should be applied in an effective amount sufficient to exert the desired biocidal activity by techniques well-known in the art. Usually, this will involve the application of an effective amount of the alpha-halopyruvate oxime to the locus to be protected incorporated in an agronomically acceptable carrier. However, in certain situations, it may be desirable and advantageous to apply the compounds directly onto the locus to be protected without the benefit of any substantial amount of carrier. This is a particularly effective method when the physical nature of the alpha-halopyruvate oxime is such as to permit what is known as "low-volume" application; that is, when the compounds are in liquid form or substantially soluble in higher boiling solvents.

The application rate will vary depending upon the purpose for such application, thealpha-halopyruvate oxime being utilized, the frequency of dissemination and the like.

For use as agricultural bactericides and fungicides, dilute sprays can be applied at concentrations of about 0.023 to 9.1 kg (0.05 to 20 pounds) of the active alpha-halopyruvate oxime ingredient per 378.5 l (100 gallon) of spray. They are usually applied at from about 0.045 kg to 4.54 kg (0.1 to 10 pounds) per 378.5 l (100 gallons) and preferably between 0.057 to 2.27 kg (0.125 to 5 pounds) per 378.5 l (100 gallons). In more concentrated sprays, the active ingredient is increased by a factor of 2 to 12. With dilute sprays, applications are usually made to the plants until run-off is achieved; whereas, with more concentrated or low-volume sprays, the materials are applied as mists.

As a fungicidal seed protectant, the amount of toxicant coated on the seed is usually at a dosage rate of

from about 0.1 to about 20 ounces per hundred pounds of seed. As a soil fungicide the chemical can be incorporated in the soil or applied to the surface usually at a rate of from about 0.112 to 56 kg/ha (0.1 to 50 lbs. per acre). As a foliar fungicide, the toxicant is usually applied to growing plants at a rate of from about 0.28 to 11.2 kg/ha (0.25 to about 10 lbs. per acre).

The composition can also be added to transplant water or employed as dips or soaks for vegetative parts employed in propagation, such as seeds, tubers, roots, seedlings, and the like, so as to disinfect and/or provide residual protection from nematodes, soil insects (and mites) and via systemic uptake, foliar pests. The application rate can be from about 0.56 to 56 kg/ha (0.5 to about 50 pounds per acre); however, higher rates can also be used. The preferred rate is from about 1.12 to 28 kg/ha (1 to about 25 pounds per acre). For soil incorporation, the compounds of this invention can be mixed with the soil at a rate of about 1 to about 100 ppm of activate ingredient.

The compounds of this invention may be utilized as the sole biocidal agents or they may be employed in conjunction with other fungicides, bactericides, algaecides, slimicides, insecticides, miticides, or with other comparable pesticides.

The following examples illustrate this invention:

EXAMPLE 1 - Ethyl 3-Bromo-2-(4-chlorobenzoyloximino)propanoate

To the mixture of ethyl 3-bromo-2-(hydroxyimino)propanoate (3 g, 14.3 mmole) and 4-chlorobenzoyl chloride (2.75 g, 15.71 mmole) in dry methylene chloride (100 ml) at 0° to 5°C with stirring, was slowly added dry pyridine (1.2 g, 15.18 mmole). After addition, the reaction mixture was stirred at room temperature for one hour. The reaction mixture was diluted with 50 ml of methylene chloride and washed with water and brine. The organic layer was dried with magnesium sulfate and the solvent evaporated to afford 5.0 g (99% yield) of ethyl 3-bromo-2-(4-chlorobenzoyloximino)propanoate as a white solid, m.p. 82°-84.5°C.

EXAMPLE 2 - Ethyl 3-Bromo-2-(methacryloyloximino)propanoate

Ethyl 3-bromo-2-(hydroxyimino) propanoate (4.0 g, 19 mmole) and methacryloyl chloride (2.18 g, 20.8 mmole) in methylene chloride (100 ml) were treated with dry pyridine (1.66 g, 21 mmole). After a workup as in Example 1, 4.42 g (84% yield) of ethyl 3-bromo-2-(methacryloyloximino)propanoate was obtained as an oil. NMR and IR spectra confirm the structure.

EXAMPLE 3 -Ethyl 3-Bromo-2-(N'-methylcarbamoyloximino)propanoate

To a solution of ethyl 3-bromo-2-(hydroxyimino)propanoate (4.0 g, 19 mmole) in methylene chloride (25 ml) was added at room temperature, methylisocyanate (5.12 g, 95 mmole, 5 equivalents) and 15 drops of triethylamine. The reaction mixture was stirred at room temperature for 48 hours. The volatiles were evaporated under reduced pressure to afford 5.56 g of ethyl 3-bromo-2-(N'-methylcarbamoyloximino)propanoate as a light yellow solid, m.p. 90°-96°C.

EXAMPLE 4 - Ethyl 3-Bromo-2-(4-methylbenzoyloximino)propanoate

To a mixture of ethyl 3-bromo-2-(hydroxyimino)propanoate (4 g, 19 mmole), and 4-methylbenzoyl chloride (3.23 g, 20.9 mmole) in dry methylene chloride (80 ml) at 0° to 5°C with stirring, was added dry pyridine (1.65 g, 20.9 mmole) from a syringe. After addition, the reaction mixture was stirred at room temperature for two more hours The reaction mixture was diluted with 50 ml of methylene chloride and washed with water and brine. The organic layer was dried over magnesium sulfate The solvent was evaporated to afford 5.51 g (88% yield) of ethyl 3-bromo-2-(4-methylbenzoyloximino)propanoate as a white solid, m.p. 94°-96°C.

By following substantially the procedure described in Examples 1 - 4 and by substituting the appropriate starting material, the following compounds are prepared. The following formula taken together with Table I illustrates the compound. Table II is a list of the NMR data for the products of Examples 5-39.

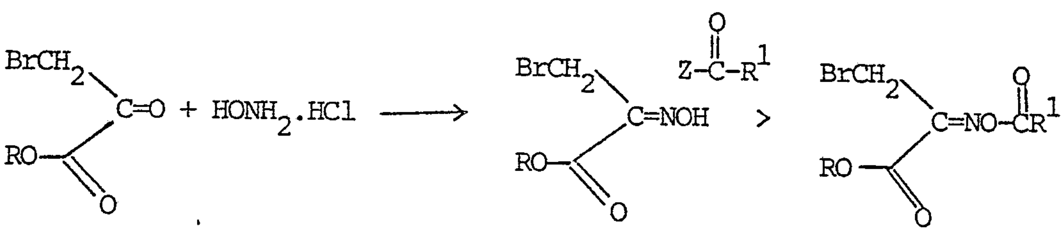

Table I

| Example No. | R | $R^1$ | Physical Data |
|---|---|---|---|
| 5 | Et | $-CH_2CH_3$ | oil |
| 6 | Et | $-OCH_3$ | oil |
| 7 | Et | $-CH=CH-C_6H_5$ | mp 113°-115°C |
| 8 | Et | $-CH_2Cl$ | oil |
| 9 | Et | -cyclohexyl | oil |
| 10 | Et | $-CH_2C_6H_5$ | oil |
| 11 | Et | 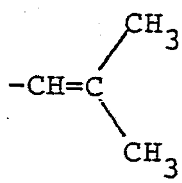 | oil |
| 12 | Et | $-C_{11}H_{23}$-<u>n</u> | oil |
| 13 | Et | $-CH_2CH_2C_6H_5$ | oil |
| 14 | Et | 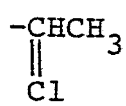 | oil |
| 15 | Et | $-CH_2CH_2Cl$ | oil |
| 16 | Et | $C_6H_4-4-CH_3$ | oil |
| 17 | Et | $-CH_3$ | oil |
| 18 | Et | $-CH=CH_2$ | oil |
| 19 | Et | $-CH=CHCH_3$ | oil |
| 20 | Et | $-C_6H_4-4F$ | mp 90°-94°C |
| 21 | Et | $-C_6H_4-2CH_3$ | oil |
| 22 | Et | $-C_3H_7$-<u>n</u> | oil |

Table I ctd

| Example No. | R | $R^1$ | Physical Data |
|---|---|---|---|
| 23 | Et | $-C_4H_9$-_n_ | oil |
| 24 | Et | $-C_6H_4-3CH_3$ | mp 56°-59°C |
| 25 | Et | $-C_5H_{11}$-_n_ | oil |
| 26 | Et | $-C_6H_4-4OCH_3$ | mp 65°-75°C |
| 27 | Et | $-C_6H_4-3OCH_3$ | oil |
| 28 | Et | $-C_6H_4-3CF_3$ | oil |
| 29 | Et | $-C_6H_4-4NO_2$ | mp 119°-124°C |
| 30 | Et | $-C_6H_5$ | mp 87°-89°C |
| 31 | Et | $-C_6H_4-4Br$ | mp 95°-98°C |
| 32 | $CH_3$ | $-C_6H_4 4Cl$ | - |
| 33 | $CH_3$ | $-C_6H_4-4-CH_3$ | - |
| 34 | Et | $-C_6H_{13}$-_n_ | oil |
| 35 | Et | $-C_{15}H_{31}$-_n_ | mp 42°-45°C |
| 36 | Et | $-C_6H_4-3Cl$ | mp 95-97°C |
| 37 | Et | $-C_6H_4-2Cl$ | - |
| 38 | Et | $-C_6H_3-3,4Cl_2$ | mp 75°-78°C |
| 39 | Et | 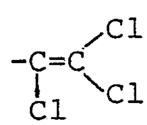 | - |
| 40 | Et | $-O-C_6H_5$ | oil |
| 41 | Et | $-O-CH_2-C_6H_5$ | mp 60°-62°C |
| 42 | Et | -cyclopropyl | oil |

Table II

NMR spectra were obtained in $CDCl_3$ solution on Varian T-60 and EM-360 instruments, with tetramethylsilane as the internal reference. Peaks positions (chemical shifts) are expressed in ppm (δ) downfield from tetramethylsilane.

| Exp. No. | Chemical Shifts (ppm or δ) |
| --- | --- |
| 5 | 4.75 to 4.22 (4H, m, $CH_2Br$ & $OCH_2$); 2.62 (2H, q, J=8Hz, $COCH_2$); 1.58 to 1.00 (6H, m, 2 $CH_3$) |
| 6 | 4.70 to 4.24 (4H, m, $CH_2Br$ & $OCH_2$); 4.00 (3H, s, $OCH_3$); 1.40 (3H, t, $CH_3$) |
| 7 | 7.52 (5H, br. s, arom. H); 7.98 & 6.64 (2H, ABq, JAB=15 Hz, vinyl H); 4.65 to 4.20 (4H, m, $CH_2Br$ & $OCH_2$); 1.38 (3H, t, $CH_3$) |
| 8 | 4.82 to 4.26 (6H, m, $CH_2Br$, $OCH_2$ & $CH_2Cl$); 1.38 (3H, t, $CH_3$) |
| 9 | 4.78 to 4.10 (4H, m, $CH_2Br$ & $OCH_2$); 2.90 to 1.00 (14H, m) |
| 10 | 7.45 (5H, s, arom. H); 4.60 to 4.15 (4H, m, $CH_2Br$ & $OCH_2$); 3.86 (2H, s, $CH_2$); 1.34 (3H, t, $CH_3$) |
| 11 | 6.02 (1H, br.s, vinyl H); 4.80 to 4.20 (4H, m, $CH_2Br$ & $OCH_2$); 2.30 (3H, s, $CH_3$); 2.10 (3H, s, $CH_3$); 1.42 (3H, t, $CH_3$) |
| 12 | 4.70 to 4.20 (4H, m, $CH_2Br$ & $OCH_2$); 2.80 to 1.10 (23H, m); 0.80 (3H, t, $CH_3$) |
| 13 | 7.30 (5H, br. s, arom. H); 4.70 to 4.20 (4H, m, $CH_2Br$ & $OCH_2$); 3.24 to 2.62 (4H, m, $CH_2CH_2$); 1.32 (3H, t, $CH_3$) |
| 14 | 5.00 to 4.14 (4H, m, $CH_2Br$ & $OCH_2$); 1.80 (3H, d, J=6 Hz, $CH_3$); 1.40 (3H, t, $CH_3$) |

| Exp. No. | <u>Chemical Shifts (ppm or δ)</u> ctd. |
|---|---|
| 15 | 5.00 to 4.20 (4H, m, $CH_2Br$ & $OCH_2$); 3.94 (2H, t, CH2Cl); 3.10 (2H, t, $COCH_2$); 1.40 (3H, t, $CH_3$) |
| 16 | 8.10 to 7.38 (4H, ABq, JAB = 8Hz, arom. H); 4.69 (2H, s, $CH_2Br$); 4.68 to 4.30 (2H, m, $OCH_2$); 2.44 (3H, s, $CH_3$); 1.40 (3H, t, $CH_3$) |
| 17 | 4.80 to 4.25 (4H, m, $CH_2Br$ & $OCH_2$); 2.30 (3H, s, $CH_3$); 1.38 (3H, t, $CH_3$) |
| 18 | 6.80 to 6.20 (3H, m, vinyl H); 4.70 to 4.20 (4H, m, $CH_2Br$ & $OCH_2$); 1.36 (3H, t, $CH_3$) |
| 19 | 7.68 to 6.98 (1H, m, <u>beta</u>-vinyl H); 6.34 to 5.90 (1H, m, <u>alpha</u>-vinyl H); 4.74 to 4.18 (4H, m, $CH_2Br$ & $OCH_2$); 2.10 to 1.76 (3H, m, $CH_3$); 1.35 (3H, t, $CH_3$) |
| 20 | 8.60 to 7.00 (4H, m, arom. H); 4.85 to 4.20 (4H, m, $CH_2Br$ & $OCH_2$); 1.42 (3H, t, $CH_3$) |
| 21 | 8.30 to 7.20 (4H, m, arom. H); 4.70 to 4.30 (4H, m, $CH_2Br$ & $OCH_2$); 2.68 (3H, s, $CH_3$); 1.40 (3H, t, $CH_3$) |
| 22 | 4.74 to 4.20 (4H, m, $CH_2Br$ & $OCH_2$); 2.54 (2H, t, $CH_2$); 2.10 to 1.50 (2H, m, $CH_2$); 1.40 (3H, t, $CH_3$); 1.02 (3H, t, $CH_3$) |
| 23 | 4.70 to 4.15 (4H, m, $CH_2Br$ & $OCH_2$); 2.58 (2H, t, $CH_2$); 2.00 to 1.20 (7H, m); 0.95 (3H, t, $CH_3$) |
| 24 | 8.20 to 7.35 (4H, m, arom. H); 4.76 to 4.28 (4H, m, $CH_2Br$ & $OCH_2$); 2.44 (3H, s, $CH_3$); 1.40 (3H, t, $CH_3$) |
| 25 | 4.74 to 4.12 (4H, m, $CH_2Br$ & $OCH_2$); 2.74 to 2.06 (2H, m, $CH_2$); 2.00 to 0.55 (12H, m) |

| Exp. No. | Chemical Shifts (ppm or $\delta$) ctd. |
|---|---|
| 26 | 8.10 to 7.02 (4H, ABq, JAB = 9Hz, arom. H); 4.80 to 4.20 (4H, m, $CH_2Br$ & $OCH_2$); 3.92 (3H, s, $OCH_3$); 1.40 (3H, t, $CH_3$) |
| 27 | 7.90 to 7.10 (4H, m, arom. H); 4.80 to 4.30 (4H, m, $CH_2Br$ & $OCH_2$); 3.95 (3H, s, $OCH_3$); 1.40 (3H, t, $CH_3$) |
| 28 | 8.80 to 7.60 (4H, m, arom. H); 4.90 to 4.30 (4H, m, $CH_2Br$ & $OCH_2$); 1.40 (3H, t, $CH_3$) |
| 29 | 8.42 (4H, br.s, arom. H); 4.80 to 4.24 (4H, m, $CH_2Br$ & $OCH_2$); 1.44 (3H, t, $CH_3$) |
| 30 | 8.70 to 7.40 (5H, m, arom. H); 4.90 to 4.20 (4H, m, $CH_2Br$ & $OCH_2$); 1.40 (3H, t, $CH_3$) |
| 31 | 8.20 to 7.56 (4H, m, arom. H); 4.80 to 4.30 (4H, m, $CH_2Br$ & $OCH_2$); 1.40 (3H, t, $CH_3$) |
| 32 | 8.04 to 7.48 (4H, ABq, JAB =8Hz, arom. H) 4.52 (2H, s, $CH_2Br$); 4.00 (3H, s, $OCH_3$) |
| 33 | 8.00 to 7.30 (4H, ABq, JAB = 8Hz, arom. H); 4.52 (2H, s, $CH_2Br$); 3.96 (3H, s, $OCH_3$); 2.42 (3H, s, $CH_3$) |
| 34 | 4.54 (2H, s, $CH_2Br$); 4.46 (2H, q, $OCH_2$); 2.84 to 2.36 (2H, m, $COCH_2$-); 2.12 to 1.16 (11H, m); 1.00 (3H, t, $CH_3$) |
| 35 | 4.68 to 4.34 (4H, m, $CH_2Br$ & $OCH_2$); 2.82 to 2.40 (2H, m, $COCH_2$-); 1.60 to 1.10 (29H, m); 0.97 (3H, t, $CH_3$) |
| 36 | 8.34 to 7.18 (4H, m, arom. H); 4.82 to 4.30 (4H, m, $CH_2Br$ & $OCH_2$); 1.52 (3H, t, $CH_3$) |
| 37 | 8.10 to 7.30 (4H, m, arom. H); 4.74 to 4.24 (4H, m, $CH_2Br$ & $OCH_2$); 1.50 (3H, t, $CH_3$) |

| Exp. No. | Chemical Shifts (ppm or δ) ctd. |
|---|---|
| 38 | 8.46 to 7.55 (3H, m, arom. H); 4.86 to 4.34 (4H, m, $CH_2Br$ & $OCH_2$); 1.56 (3H, t, $CH_3$) |
| 39 | 4.86 to 4.26 (4H, m, $CH_2Br$ & $OCH_2$); 1.52 (3H, t, $CH_3$) |
| 40 | 7.60 to 7.00 (5H, br.s, arom. H); 4.68 to 4.20 (4H, m, $CH_2Br$ & $OCH_2$); 1.34 (3H, t, $CH_3$) |
| 41 | 7.40 (5H, s, arom. H); 5.25 (2H, s, $CH_2$); 4.60 to 4.20 (4H, m, $CH_2Br$ & $OCH_2$); 1.36 (3H, t, $CH_3$) |
| 42 | 4.70 to 4.18 (4H, m, $CH_2Br$ & $OCH_2$); 2.10 to 0.85 (8H, m, $CH_3$ & cyclopropyl) |

The following test methods were employed to determine the various activities of those compounds.

I. Description of Biocide Test Procedure

1. A minimum inhibitory concentration (MIC) value is obtained using a broth, two-fold serial dilution test performed as follows: A stock solution or dispersion of the test compound, typically at a concentration of 1%, is made in an organic solvent, such as acetone. A volume of the stock solution is dispensed into culture media to give an initial starting test concentration of 250-500 ppm compound.

When the test is ready to be done, each vessel in the dilution series, except the first vessel, contains an equal volume of compound free broth. The first vessel contains twice the volume of broth with the starting concentration of test compound. One half of the broth from the first vessel is transferred to the second vessel. After being mixed, one half the resulting volume is removed from the second vessel and transferred to the third vessel. The entire cycle is repeated 8 to 12 times, depending on the number of dilutions desired. At the end of the series of dilutions, each succeeding vessel in the series has one half the concentration of test compound the previous vessel has.

Each vessel is then inoculated with a cell suspension of the appropriate test organism. Bacteria are grown in broth and fungi or agar slants, for a time and at a temperature appropriate to the species being tested. At the end of the growth period, the broth is vortexed to disperse the cells. In the case of fungi, the spores are harvested by pipetting water onto the salt and dislodging the spores with a sterile loop. The cell/spore suspension is standardized by controlling incubation time and temperature and the volume of the diluent. The suspension is then used to inoculate the vessels containing the broth compound The vessels are then incubated at the appropriate temperature. After the incubation, the vessels are examined for growth/no growth. The minimum inhibitory concentration (MIC) is defined as the lowest concentration of compound that results in complete inhibition of growth of the test organism.

2. The speed of kill test measures loss of cell viability in an aqueous suspension of bacterial cells as a function of time when these cells are contacted with a defined concentration of test compound in the water. This is done by taking aliquots of the cell suspensions at the appropriate time interval and assaying the number of viable cells per milliliter by plate count or most probable number (MPN) methodology. These measurements are done on the cell suspensions containing test compound and on control suspensions containing no test compound. The viable cell counts of the test and control samples are then compared to determine cell death.

The test is set up by first dissolving the compound in an organic solvent, such as acetone, to make up a stock solution. This stock solution is typically at 1% concentration. The stock solution is then dispensed into sterile, synthetic hard water, typically at 200 ppm hardness expressed at $CaCO_3$, to give a final concentration of test compound of 100 ppm.

The inoculum is prepared by growing the bacteria on a slant for 24 hours and then harvesting the cells into phosphate buffer. To start the test at zero time, one volume of bacterial inoculum is added to 100 volumes of test solution containing compound at the final test concentration.

At appropriate time intervals, ranging from 10 min to 24 hrs, aliqouots of all the test samples and controls are assayed for viable cell count, in colony forming units (CFU)/ml.

The results are calculated in terms of $\log_{10}$ reduction in CFU/ml compared to aqueous control. This is done by taking the logarithm base 10 of the CFU/ml for the aqueous control count. One log reduction corresponds

to 90% kill, 2 logs reduction corresponds to 99% kill, 3 logs reduction corresponds to 99.9% kill, etc.

Tested Organisms for "Biocides":

Bacteria:
Pseudomonas fluorescens
Pseudomonas aeruginosa
Staphylocoecus aureus and
Escherichia coli

Fungi:
Aspergillis niger
Candida albicans
Aureobasidium pullulans

Tested Diseases for "Fungicides":

1. Wheat Leaf Rust (puccinia graminis)
2. Tomato Late Blight (phytophthora infestans)
3. Wheat Powdery Mildew (Erysiphe graminis)
4. Cucumber Downey Mildew (Pseudoperonspora cubensis)
5. Rice sheath Blight (Rhizoctonia Solani)
6. Rice Blast (Pyricularia oryzae)

Description of Fungicides Test Method

Wheat Leaf Rust (Puccinia graminis)

Wheat seedlings cultivar 'Fielder' are grown in redi-earth and used for screening about seven days after planting. The seedlings are fertilized with liquid-M prior to use. The spore suspension is prepared by rehydrating from spores (from deep freeze (-20°C)) and adding Seltrol spray oil at a concentration of 4 mg. spores per ml oil. Inoculum is then dispensed into gelatin capsules and applied with a vacuum pump. Four passes are made on both sides of the plant for uniformity. Plants are allowed twenty minutes to dry and then placed in a humidity cabinet (100% RH) in the dark for 20-24 h at 70°C. Plants are transferred to the greenhouse and evaluated 13 days later.

Tomato Late Blight (Phytophthora infestans)

Tomato (var. Pixie) seedlings, 7.62-10.16cms (3-4 inches) tall are used for screening. A spore suspension of the fungus is obtained by adding water to a jar of sporulating tomato leaves. The spore suspension is applied with a DeVilbiss atomizer at a guage air pressure of 0.55-0.69 Bar (8 to 10 psi) onto the leaf undersurface until fine droplets are formed. Inoculated seedlings are placed in a humid environment (100% RH) at 15.56-18.33°C (60°-65°F) for 24 h, prior to being placed in a controlled temperature room in intermittent mist chambers at 20°C and 90% RH. Treatment comparisons are made 4-5 days after inoculation.

Wheat Powdery Mildew (Erysiphe graminis)

"Victory-283" wheat seedlings (7 days old) are used as test plants. Wheat powdery mildew is cultured on wheat seedlings in a controlled temperature room at 18.33-23.89°C (65°-75°F). After chemical application, mildew spheres are shaken from culture plants onto seedlings. Inoculated seedlings are kept in the controlled temperature room and subirrigated. Disease control is rated 7 days after inoculation.

Cucumber Downey Mildew (Pseudoperonspora cubensis)

Cucumber (var. "Marketer") seedlings are grown for three weeks at 65°-75°F in moderate light before use. Pseudoperonspora cubensis is cultured on cucumber seedlings for 7 days at 18.33-23.89°C (65°-75°F) in moderate light (alternating light and dark periods). Spores are harvested by adding deionized water and shaking leaves in a quart jar. The spore suspension is filtered through cheesecloth to remove plant debris and adjusted to a concentration of $1 \times 10^5$ spores per ml.

The cucumber plants are inoculated by spraying the under side of the leaves with a DeVilbiss atomizer until small droplets are on the leaves. The inoculated leaves are incubated in a mist chamber for 24 hours at 21.11°C (70°F) and then subsequently incubated for 6-7 days in a controlled temperature room under mist at 18.33-23.89°C (65°-75°F).

Treatment comparisons are made 7 days after inoculation by estimating percent infected leaf surface. Symptoms appear as yellowing of the upper leaf surface and grey sporulating areas on the lower leaf surface.

Rice Sheath Blight (Rhizoctonia solani)

Seedlings of rice cultivar M-201 are grown in the greenhouse at 20-30°C in 5.1 cm (2-inch) pots in unsterilized soil with Turf-Builder for 14 days. Prior to the application of chemicals the plants are trimmed with scissors to a height of 10.2-12.7 cm (4-5 inches).

Inoculum is produced in shake culture using the following procedure: autoclaved 500 ml wide-mouth flasks containing 150 ml potato dextrose broth are inoculated with a small piece of mycelium or a single sclerotium of Rhizotonia solani Kuhn. The flasks are placed on an electric shaker (1500 rpm) at 22° C and a photoperiod of 14-16 hrs for 6 days. A slurry containing 100 ml deionized water, 20g rice flour (no additives) and 23g mycelium (wet weight) is prepared in a blender in the appropriate quantity to inoculate 5.1 cm (2 inch) pots with 4 ml/pot. Blend the mixture for about 1 min.

The slurry is dispensed into 5.1 cm (2 inch) pots using pipettes with an oversized opening at 4 ml/pot (10 ml per 7.62 cm (3 inch) pot). While dispensing the inoculum, the pot should be tilted to insure uniform distribution of the slurry over the entire soil surface. During the inoculation the slurry is kept in suspension using a stirring plate at medium speed (high speed causes foaming).

Plants are put in a humidity cabinet at 28° C for 43 hrs and then kept in a humidity cabinet at 25° C for 53 hrs (photoperiod for both cases 16 hrs). The height of mycelial growth is observed as compared to the inoculated control plants. Records are kept as % control.

Rice Blast foliar treatments (Pyricularia oryzae)

Seedlings of the rice cultivar 'M-201' are grown in the greenhouse at 20°-30° C in 5.1 cm (2-inch) pots containing unsterilized soil+Turf-Builder for 14 days. Rice plants are not trimmed before use.

Inoculum is produced in-vitro on oatmeal agar (50g Gerber baby oatmeal, 20g bacto agar, 10g bacto Dextrose, 1000ml deionized water). The plates are inoculated with a mycelial plug (7-14 days old) of Piricularia oryzae. The outer edge of the dark region is used in the transfer. Inoculated plates are maintained at room temperature under constant fluorescent light.

Pyricularia oryzae plates 10-14 days old are flooded with a solution containing: 0.25g Sodium oleate, 2g gelatin, 1000ml deionized water. The plates are scraped with a rubber policeman (a miniature rubber spatula) to release conidia, filter through a double layer of cheesecloth and adjust spore suspension of 25000-30000 spores/ml using a hemacytometer.

The spore suspension is sprayed on opposite sides of a double row of rice plants using a hand sprayer. Sufficient inoculum should be applied to achieve uniform distribution from soil to tip of rice leaves on opposite sides of each pot (approx. 50 ml/50 pots). Shake the sprayer after each pass to keep solution in suspension.

Inoculated plants are immediately placed in a humidity cabinet at 25° C for 66 hrs prior to moving them to greenhouse bay 4 under the plastic tent. Plants are subirrigated but not allowed to stand in water more than 2 hrs. The plastic sides are lifted during work hrs and always closed at end of day.

After 76 hrs under greenhouse condition the bioassay plants are observed and the percent disease control (as compared to inoculated control) is estimated.

## Claims

1. A method of controlling fungi or bacteria which comprises applying, to the fungi or bacteria or to a locus which is to be protected from or is under bacterial or fungal attack, an effective amount of a compound of the formula:

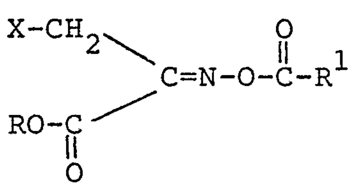

wherein X is halo; R is alkyl; $R^1$ alkyl, haloalkyl, alkenyl, haloalkenyl, alkoxy, haloalkoxy, alkoxycarbonyl, alkoxycarbonylalkyl, aryl, aryloxy, aryloxy- alkyl, aralkyl, aralkenyl, cycloalkyl, substituted aryl, substituted aryloxy or substituted aryloxyalkyl (wherein the substituent is selected from one or more halo, $(C_1\text{-}C_6)$alkyl, $(C_1\text{-}C_6)$alkoxy, halo-$(C_1\text{-}C_6)$alkyl, or nitro substituents) or $(C_1\text{-}C_6)$alkylamino.

2. A method according to claim 1 wherein the compound is of the following formula:

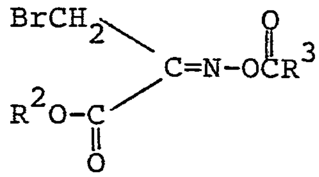

wherein $R^2$ is $(C_1\text{-}C_6)$alkyl and $R^3$ is halo-$(C_1\text{-}C_6)$alkyl, $(C_1\text{-}C_6)$alkoxy, $(C_2\text{-}C_6)$alkenyl, phenyl-$(C_1\text{-}C_6)$alkyl, halophenyl, trifluoromethylphenyl, nitrophenyl or methylamino.

3. A method according to claim 2 wherein $R^2$ is methyl or ethyl and $R^3$ is chloromethyl, methyl, ethyl, propyl, isopropyl, butyl, 1-methylethenyl, methoxy, 2-, 3- or 4-chlorophenyl, 2-, 3-, or 4-methyl phenyl, 2-, 3-, or 4-fluorophenyl, 2,4- or 2,6-dichlorophenyl, ethenyl, 1-propenyl, chloromethyl or 1-phenylethenyl.

4. A method according to any preceding claim wherein the compound is incorporated into a composition wherein attack is to be inhibited.

5. A method according to claim 4 wherein the compound is incorporated into the composition in an amount of from 0.1 to 10,000 parts per million, preferably 0.05 to 2,500 parts per million.

6. A compound of the formula

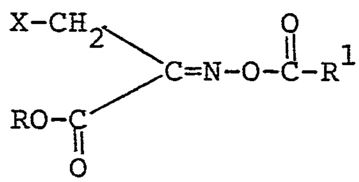

wherein X is halo; R is alkyl and $R^1$ is haloalkyl, haloalkenyl, haloalkoxy, $(C_1\text{-}C_6)$alkylamino or aryl substituted with one or more halo, haloalkyl or nitro.

7. A compound according to claim 6 wherein X is bromine, R is $(C_1\text{-}C_{15})$alkyl and $R^1$ is halo-$(C_1\text{-}C_6)$alkyl, halophenyl, trifluoromethylphenyl, nitrophenyl or methylamino.

8. A compound according to claim 6 or 7 wherein R is methyl or ethyl and/or $R^1$ is chloromethyl, 2-, 3- or 4-chlorophenyl, 2-, 3- or 4-methylphenyl, 2-, 3- or 4-fluorophenyl or 2,4- or 2,6-dichlorophenyl.

9. A compound according to any of claims 6 to 8 or a method according to any of claims 1 to 5 wherein X is bromine, R is ethyl and $R^1$ is 4-chlorophenyl or 4-methylphenyl or 3-methylphenyl or propyl or 1-methylethenyl or 2-phenylethenyl or 4-fluorophenyl.

10. A bactericidal and/or fungicidal composition comprising a compound according to any of claims 6 to 9 together with environmentally acceptable diluent or carrier.

11. A method according to claim 1, 4 or 5 wherein the compound is as claimed in any of claims 6 to 9.

Claims for the following Contracting State : AT

1. A method of controlling fungi or bacteria which comprises applying, to the fungi or bacteria or to a locus which is to be protected from or is under bacterial or fungal attack, an effective amount of a compound of the formula:

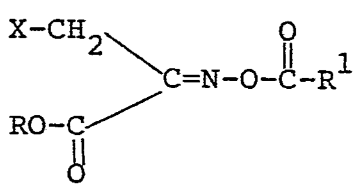

wherein X is halo; R is alkyl; $R^1$ alkyl, haloalkyl, alkenyl, haloalkenyl, alkoxy, haloalkoxy, alkoxycarbonyl, alkoxycarbonylalkyl, aryl, aryloxy, aryloxy- alkyl, aralkyl, aralkenyl, cycloalkyl, substituted aryl, substituted aryloxy or substituted aryloxyalkyl (wherein the substituent is selected from one or more halo, $(C_1\text{-}C_6)$alkyl, $(C_1\text{-}C_6)$alkoxy, halo-$(C_1\text{-}C_6)$alkyl, or nitro substituents) or $(C_1\text{-}C_6)$alkylamino.

2. A method according to claim 1 wherein the compound is of the following formula:

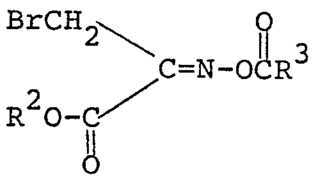

wherein $R^2$ is $(C_1\text{-}C_6)$alkyl and $R^3$ is halo-$(C_1\text{-}C_6)$alkyl, $(C_1\text{-}C_6)$alkoxy, $(C_2\text{-}C_6)$alkenyl, phenyl-$(C_1\text{-}C_6)$alkyl, halophenyl, trifluoromethylphenyl, nitrophenyl or methylamino.

3. A method according to claim 2 wherein $R^2$ is methyl or ethyl and $R^3$ is chloromethyl, methyl, ethyl, propyl, isopropyl, butyl, 1-methylethenyl, methoxy, 2-, 3- or 4-chlorophenyl, 2-, 3-, or 4-methyl phenyl, 2-, 3-, or 4-fluorophenyl, 2,4- or 2,6-dichlorophenyl, ethenyl, 1-propenyl, chloromethyl or 1-phenylethenyl.

4. A method according to any preceding claim wherein the compound is incorporated into a composition wherein attack is to be inhibited.

5. A method according to claim 4 wherein the compound is incorporated into the composition in an amount of from 0.1 to 10,000 parts per million, preferably 0.05 to 2,500 parts per million.

6. A bactericidal and/or fungicidal composition comprising a compound of the formula

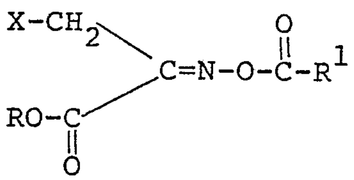

wherein X is halo; R is alkyl and $R^1$ is haloalkyl, haloalkenyl, haloalkoxy, $(C_1\text{-}C_6)$alkylamino or aryl substituted with one or more halo, haloalkyl or nitro;
together with environmentally acceptable diluent or carrier.

7. A composition according to claim 6 wherein X is bromine, R is $(C_1\text{-}C_{15})$alkyl and $R^1$ is halo-$(C_1\text{-}C_6)$alkyl, halophenyl, trifluoromethylphenyl, nitrophenyl or methylamino.

8. A composition according to claim 6 or 7 wherein R is methyl or ethyl and/or $R^1$ is chloromethyl, 2-, 3- or 4-chlorophenyl, 2-, 3- or 4-methylphenyl, 2-, 3- or 4-fluorophenyl or 2,4- or 2,6-dichlorophenyl.

9. A composition according to any of claims 6 to 8 or a method according to any of claims 1 to 5 wherein X is bromine, R is ethyl and $R^1$ is 4-chlorophenyl or 4-methylphenyl or 3-methylphenyl or propyl or 1-methylethenyl or 2-phenylethenyl or 4-fluorophenyl.

10. A method according to claim 1, 4 or 5 wherein the compound is used in a composition as claimed in any of claims 6 to 9.

Claims for the following Contracting State : ES

1. A process for controlling fungi or bacteria which comprises applying, to the fungi or bacteria or to a locus which is to be protected from or is under bacterial or fungal attack, an effective amount of a compound of the formula:

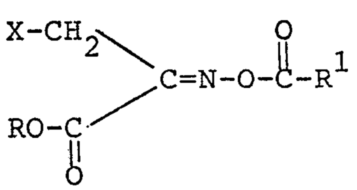

wherein X is halo; R is alkyl; $R^1$ alkyl, haloalkyl, alkenyl, haloalkenyl, alkoxy, haloalkoxy, alkoxycarbonyl, alkoxycarbonylalkyl, aryl, aryloxy, aryloxy- alkyl, aralkyl, aralkenyl, cycloalkyl, substituted aryl, substituted aryloxy or substituted aryloxyalkyl (wherein the substituent is selected from one or more halo, $(C_1\text{-}C_6)$alkyl, $(C_1\text{-}C_6)$alkoxy, halo-$(C_1\text{-}C_6)$alkyl, or nitro substituents) or $(C_1\text{-}C_6)$alkylamino.

2. A process according to claim 1 wherein the compound is of the following formula:

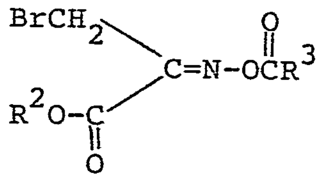

wherein $R^2$ is$(C_1-C_6)$alkyl and $R^3$ is halo-$(C_1-C_6)$alkyl, $(C_1-C_6)$alkoxy, $(C_2-C_6)$alkenyl, phenyl-$(C_1-C_6)$alkyl, halophenyl, trifluoromethylphenyl, nitrophenyl or methylamino.

3. A process according to claim 2 wherein $R^2$ is methyl or ethyl and $R^3$ is chloromethyl, methyl, ethyl, propyl, isopropyl, butyl, 1-methylethenyl, methoxy, 2-, 3- or 4-chlorophenyl, 2-, 3-, or 4-methyl phenyl, 2-, 3-, or 4-fluorophenyl, 2,4- or 2,6-dichlorophenyl, ethenyl, 1-propenyl, chloromethyl or 1-phenylethenyl.

4. A process according to any preceding claim wherein the compound is incorporated into a composition wherein attack is to be inhibited.

5. A process according to claim 4 wherein the compound is incorporated into the composition in an amount of from 0.1 to 10,000 parts per million, preferably 0.05 to 2,500 parts per million.

6. A mechanical process for improving the commercial value and/or profitability of vendible crops from plants whose growth is affected or likely to be affected by bacteria or fungi which comprises (1) charging to a container, fumigation device or mechanical dissemination device a bactericidal and/or fungicidal composition containing a compound as defined in any preceding claim (2) using the container fumigator or mechanical dissemination device to apply the bactericidal and/or fungicidal composition, in the form of granules, dust, smoke, vapour or surfactant-containing liquid preparation to growing plants or to a growth medium where the plants are growing or are to be grown, or to the bacteria or fungi themselves (3) controlling the dose of the active ingredient during this application step so that the rate of application of active bactericidal and/or fungicidal compound is sufficient to combat the bacteria or fungi but is insufficient to cause an unacceptably adverse effect on the crop plants growing or to be grown in the treated area.

7. The use of a compound of the formula

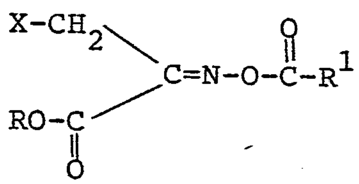

wherein X is halo; R is alkyl and $R^1$ is haloalkyl, haloalkenyl, haloalkoxy, $(C_1-C_6)$alkylamino or aryl substituted with one or more halo, haloalkyl or nitro, together with environmentally acceptable diluent or carrier, to make a bactericidal and/or fungicidal composition.

8. The use according to claim 7 of a compound wherein X is bromine, R is $(C_1-C_{15})$alkyl and $R^1$ is halo-$(C_1-C_6)$-alkyl, halophenyl, trifluoromethylphenyl, nitrophenyl or methylamino.

9. The use according to claim 7 or 8 of a compound wherein R is methyl or ethyl and/or $R^1$ is chloromethyl, 2-, 3- or 4-chlorophenyl, 2-, 3- or 4-methylphenyl, 2-, 3- or 4-fluorophenyl or 2,4- or 2,6-dichlorophenyl.

10. The use according to any of claims 7 to 8 of a compound, or a method according to any of claims 1 to 5, wherein X is bromine, R is ethyl and $R^1$ is 4-chlorophenyl or 4-methylphenyl or 3-methylphenyl or propyl or 1-methyl- ethenyl or 2-phenylethenyl or 4-fluorophenyl.